# EUROPÄISCHE PATENTSCHRIFT

(11) **EP 0 787 291 B1**
(45) Veröffentlichungstag und Bekanntmachung des Hinweises auf die Patenterteilung: **12.08.1998**
(21) Anmeldenummer: 95935950.6
(22) Anmeldetag: 19.10.1995
(51) Int. Cl.: G01N 27/12

(54) **CHEMISCHER FESTKÖRPERSENSOR**
SOLID-STATE CHEMICAL SENSOR
DETECTEUR A SEMI-CONDUCTEUR

(30) Priorität: 21.10.1994 DE 4437692
(43) Veröffentlichungstag der Anmeldung: 06.08.1997
(73) Patentinhaber: FRAUNHOFER-GESELLSCHAFT ZUR FÖRDERUNG DER ANGEWANDTEN FORSCHUNG E.V., 80636 München (DE)
(72) Erfinder: MEYER, Jörg-Uwe, D-66386 St. Ingbert (DE); HAEUSLER, Andrea, D-66121 Saarbrücken (DE)
(86) Internationale Anmeldenummer: EP9504108
(87) Internationale Veröffentlichungsnummer: WO9612944

(56) Entgegenhaltungen:
- EP-A- 0 488 503
- PATENT ABSTRACTS OF JAPAN vol. 17 no. 522 (P-1616) ,20.September 1993 & JP,A,51 042180 (YONETANI KAZUHIRO) 8.Juni 1993,
- THIN SOLID FILMS, Nr. 245, Juni 1994 LAUSANNE, Seiten 225-227, XP 000453832 BEIPING YAN ET AL. 'Gas-sensing properties of alfa-Fe2O3 thin Films prepared by plasma-enhanced chemical vapour deposition'

## Beschreibung

### Technisches Anwendungsgebiet

Die Erfindung betrifft einen Chemischen Festkörpersensor nach dem Oberbegriff des Anspruchs 1, wie er z.B. aus Technisches Messen, tm 56 (1989) Oldenbourg-Verlag, Seiten 260- 63, bekannt ist.

Die Detektion und Kontrolle der Konzentration von Kohlendioxid erlangt jedoch eine immer weiter wachsende Bedeutung in industriellen und biologischen Prozessen. Als Schadstoff wurde dem CO₂ erst in den letzten Jahren durch die steigende Aufmerksamkeit auf den sogenannten Treibhauseffekt genügend Bedeutung beigemessen.

Weitere Einsatzgebiete der Chemischen Festkörpersensoren sind:
- Raumluftüberwachung und Steuerung von Klima- und Belüftungsanlagen;
- Klimaüberwachung in Gewächshäusem und anderen Bereichen der Landwirtschaft;
- Biotechnologie, Kontrolle biologischer und chemischer Prozesse;
- Patientenmonitoring;
- kontinuierliche und umfassende Kontrolle der Schadstoffemission in industriellen Verbrennungsprozessen.

Die sich international verschärfende Verordnungslage und das zunehmende Umweltbewußtsein in der Bevölkerung lassen einen zunehmenden Bedarf an preisgünstigen CO₂-Sensoren erwarten.

### Stand der Technik; Nachteile des Standes der Technik

Zur Detektion von Kohlendioxid werden ganz unterschiedliche Sensormaterial-Eigenschaften bzw. deren Änderung unter Einwirkung des zu detektierenden Gases ausgenutzt.

### Festelektrolyt-Sensoren

Ein nach diesem Prinzip arbeitender Sensor zur Detektion von Kohlendioxid wurde u.a. von T. MARUYAMA und Mitarbeitem (Tokyo Institute of Technology, Japan) entwickelt. Er basiert auf dem Na⁺-Ionenleiter NASICON. Daran angebracht ist eine Karbonat-Elektrode, die auf CO₂-Änderungen anspricht [1-3]. Während der Zufuhr von Kohlendioxid bilden sich an der Anode Natrium-Ionen aus. Diese wandem durch das Karbonat zum Elektrolyten und erreichen die Kathode. Die auftretende elektromotorische Kraft (EMK) wird gemessen und anhand der NERNST-Gleichung die CO₂-Konzentration in der Probe bestimmt. Lange Antwortzeiten, da die Änderungsgeschwindigkeit der EMK von der Diffusionsgeschwindigkeit des die Leitfähigkeit im Elektrolyten erzeugenden Ions abhängt, und Beeinflussung des Meßergebnisses durch Feuchtigkeit sind große Nachteile dieser Sensoren. Einen weiteren Nachteil stellt die Notwendigkeit einer Referenzelektrode dar, da diese von der Umgebung gasdicht isoliert werden muß. Die Problematik einer stabilen Referenzelektrode konnte bisher nicht zufriedenstellend gelöst werden.

### Optische CO₂-Sensoren

Eine vielfach zur Detektion von CO₂ angewandte Meßmethode ist die Infrarot-Absorption. Diese nicht-destruktive Technik basiert auf der Fähigkeit der CO₂-Moleküle, Infrarotstrahlung bei Wellenlängen zu absorbieren, die für die chemische Struktur der Moleküle spezifisch sind. Spektrometer zeigen eine große Stabilität und Genauigkeit. Ein hoher Entwicklungsstand ist bis heute erreicht worden, dennoch sind solche Geräte sehr teuer und erfordem zumeist einen großen, apparativen Aufwand. Diese Analysentechnik kommt daher aus Preisgründen für Überwachungsaufgaben im Feld nicht in Betracht.

Bei anderen optischen CO₂-Sensoren ist die Änderung der Brechzahl eine Funktion der CO₂-Konzentration. Am Fraunhofer Institut für Physikalische Meßtechnik in Freiburg unter R. EDELHÄUSER wurde ein integrierter optischer Sensor entwickelt, bei dem organische, modifizierte Silikate als empfindlicher Film auf einem integrierten optischen Interferometer aufgebracht wurden [4]. Das an diesem Film absorbierende CO₂ bewirkt, daß sich dessen Brechungsindex ändert. Probleme mit der Selektivität, der Zeitkonstanten und der Reversibilität konnten bisher nicht gelöst werden.

Eine weitere optische Meßmethode ist die Verwendung eines geeigneten Indikators, der unter Einwirkung des CO₂ seine Farbe ändert. Die Intensität dieser Farbänderung ist von der Konzentration des Gases abhängig und wird optisch ausgewertet. Auch hier wird an einer integrierten Lösung gearbeitet (C.H. MORGAN, Microsensor Research Laboratory, University of Washington, USA) [5].

### Massensensitive Sensoren

Gravimetrische Sensoren adsorbieren die zu messenden Gase an ihrer Oberfläche reversibel. Durch Anlagerung der zu untersuchenden Substanz an der Sensoroberfläche kommt es zu einer Masseänderung und damit zu einer Änderung der Ausbreitungsgeschwindigkeit der Welle bzw. Verschiebung der Resonanzfrequenz.

An der Universität Tübingen wurde in der Gruppe von Prof. W. GÖPEL ein Quartz Microbalance Sensor mit einem auf Silicon basierenden Polymer beschichtet, um Kohlendioxid zu detektieren [6].

Von NIEUWENHUIZEN et al. (Prins Maurits Laboratory TNO, Rijswijk, Niederlande) wurde Poly(ethyleneimim) als chemisches Interface für CO₂ auf einen Surface Acoustic Wave-Sensor aufgebracht [7]. Es zeigte sich jedoch, daß die starken Querempfindlichkeiten von Wasser und Sauerstoff den Sensor für CO₂-Messungen unbrauchbar machen. Gleichzeitig nahm die CO₂-Empfindlichkeit mit zunehmender Betriebsdauer ab und es war eine starke Baseline-Drift zu beobachten.

Die charakteristische Frequenz ist bei dieser Sensorgruppe sowohl deutlich temperatur-als auch druckempfindlich. Im gegenwärtigen Stand der Entwicklung sind solche Sensor-typen weniger zur CO₂-Detektion geeignet.

### Ultraschall-Sensor

Bei diesem Sensortyp, der von V.M. MECEA (Institute of Isotopic and Molecular Technology, Cluj-Napoca, Rumänien) entwickelt wird, generiert ein Quarzkristall-Resonator Ultraschallwellen in einem Hohlraum, die von dessen Wänden reflektiert werden. Ist der Abstand zwischen der Quarz-Oberfläche und den parallelen, reflektierenden Wänden ein ganzzahliges Vielfaches der halben Wellenlänge, dann tritt Resonanz mit dem sich im Hohlraum befindenden Gas auf und die gesamte Schwingungsenergie des Resonators wird vom Gas absorbiert. Durch Einstellen des Spaltes auf eine bestimmte Länge, kann die Resonanz von CO₂ erhalten werden. Befindet sich ein kleiner Anteil eines anderen Gases im Gasfluß, so ändert sich die Resonanzbedingung und kann als Signal ausgewertet werden. [8] Die Nachteile dieses Sensors sind die starke Temperaturdrift und die bisher nicht vorhandene Selektivität.

### Kapazitive Meßverfahren

Die Dielektrizitätskonstante des Sensormaterials ändert sich dadurch, daß Moleküle mit geeignetem Dipolmoment an der Sensoroberfläche adsorbiert werden. CO₂ besitzt kein eigenes Dipolmoment. Aus diesem Grund werden für kapazitive CO₂-Sensoren Materialien verwendet, an die das Kohlendioxid chemisorbieren kann, so daß das Endprodukt eine Veränderung der Dielektrizitätskonstante hervorruft. Von der CO₂-Konzentration in der Umgebung des Sensors ist es abhängig, in welchem Grad die Adsorption stattfindet und folglich auch die Größe der Änderung der Dielektrizitätskonstante. Diese Änderung kann mit einer Kondensatorstruktur ermittelt werden.

Unter E. OBERMEIER (TU Berlin) wird CO₂-empfindliches Material auf der Basis organischer, modifizierter Silikate auf mittels Dünnfilmtechnik hergestellten Interdigitalkondensatoren aufgebracht. Die dynamischen Sensoreigenschaften sind extrem temperaturabhängig. Gleichzeitig weist der Sensor eine hohe Feuchteempfindlichkeit auf. [9,10] Forschungsarbeiten hinsichtlich eines kapazitiven Meßverfahrens zur Bestimmung der CO₂-Konzentration auf der Basis von Metalloxiden wurden von einer Gruppe um T. ISHIHARA an der Oita Universität (Japan) veröffentlicht. Die Grundidee dieser Sensoren basiert darauf, daß sich die Dielektrizitätskonstanten der Metalloxide prinzipiell von denen der Metallkarbonate unterscheiden [11-14]. Ein aus einem Gemisch verschiedener Metalloxide bestehendes Pulver wurde zu Tabletten gepreßt und gesintert. Durch Aufbringen von Silber auf beiden Seiten der Tablette wurden die Elektroden hergestellt, um so eine Kondensatorstruktur zu erhalten. In die Silbertropfen gesteckte Drähte stellen die Kontaktierung dar. Eine Erhöhung der CO₂-Konzentration in der Umgebung führt zu einem Anstieg der Sensorkapazität. Es tritt gleichsam ein Einfluß von Wasser aufgrund seiner großen Dielektrizitätskonstante auf. Dieser Aufbau der CO₂-Sensoren stellt sich als sehr nachteilig dar. Tabletten sind im Einsatz in der Sensorik unhandlich, da sie leicht brechen. Aufgrund der Kontaktierungsschwierigkeiten bei aus Pulver gepreßten Tabletten, selbst mit Sputtem aufgebrachte Elektroden wären wegen ihrer notwendigen geringen Schichtdicke nicht löt- oder bondbar, wird auf das wenig reproduzierbare Auftropfen von Silber zur Herstellung der Elektroden zurückgegriffen. Die Elektrodenfläche und - form sind nicht reproduzierbar und ungenau in ihrer Fertigung, so daß die Sensorkapazität als eine Funktion der Elektrodenfläche ebenfalls nicht reproduzierbar ist. Gleichsam wird die chemisch aktive Schicht durch die Elektroden von der Umgebung weitgehend abgeschirmt. Durch den durch die Dicke der Tablette bestimmten großen Elektrodenabstand von 0,6 mm besitzt der Sensor eine hohe Impedanz. Das Verhältnis zwischen Volumen und Oberfläche des Sensorkörpers ist sehr groß, der Diffusionsweg lang. Die Einstellung der zur CO₂-Detektion erforderlich hohen Temperatur des Sensormaterials von ca. 475°C muß über eine exteme Heizvorrichtung erzielt werden. Um die Änderungen der dielektrischen Eigenschaften zu erfassen, ist eine komplizierte Auswerteschaltung erforderlich. Sensoren in Tablettenform mit der oben dargestellten Art der Kontaktierung sind für eine Massenfertigung nicht geeignet.

### Polymer-Leitfähigkeitssensoren

Das Prinzip dieser Sensoren beruht auf einer Leitfähigkeitsänderung, die durch Gas-adsorption und die sich anschließende Oberflächenreaktion hervorgerufen wird. Eine gewisse Selektivität wird durch die Wahl des Katalysators erreicht. [15] Erste Untersuchungen an Polymer-Leitfähigkeitssensoren wurden an Sensoren auf der Grundlage von PPA (Polyphenylacetylen) [16] durchgeführt. Dieses Sensorprinzip konnte bisher nicht erfolgreich in die Praxis umgesetzt werden.

Aufgabe der Erfindung ist es, den Chemischen Festkörpersensor so auzubilden, daß keine Referenzelektrode nötig ist und die Sensor wesentlich billiger wird, so daß er die Möglichkeit bietet, mit relativ geringen Kosten engmaschig Schadstoffkonzentrationen in der Industrie, am Arbeitsplatz oder im Straßenverkehr mit ausreichender Genauigkeit zu ermitteln. Die Kontrolle der CO₂-Konzentration in der Umgebung macht es dann möglich, Umweltgefahren quantitativ zu erfassen und gezielte Gegenmaßnahmen einzuleiten.

Erfindungsgemäß wird dies mit einem Chemischen Festkörpersensor gemäß Anspruch 1 gelöst. Vorteilhafte Ausgestaltungen des Sensors sind in den Unteransprüchen gekennzeichnet.

### Mit der Erfindung gelöste Aufgabe

Es wurde ein Dickschicht-Metalloxid-Leitfähigkeitssensor entwickelt, der auf eine Veränderung der Kohlendioxid-Konzentration in seiner Umgebung mit einer Erhöhung bzw. Verringerung seines Widerstandes antwortet. Aufgrund seiner Funktionsweise, der Änderung seiner Leitfähigkeit infolge einer CO₂-Konzentrationsänderung, ist der Einsatz einer einfachen Elektronikschaltung zur Auswertung des Sensorsignals möglich.

Da zur CO₂-Konzentrationsbestimmung der Sensorwiderstand ausgewertet wird, besitzt der Sensor eine sehr einfache Struktur und kann mittels Dickschichttechnik gefertigt werden. Damit ist es gelungen, CO₂-Leitfähigkeitssensoren in einem Verfahren herzustellen, das sich angesichts seiner Einfachheit, seiner großen industriellen Verbreitung, seinem geringen apparativen Aufwand, seiner niedrigen Investitionskosten und seiner guten Wirtschaftlichkeit zur Massenproduktion eignet.

Durch die Wahl der Ausgangsmaterialien konnte nicht nur ein gutes Ansprechen auf Veränderungen der CO₂-Konzentration erzielt werden sondem auch, trotz des Weglassens der in der Dickschichttechnik gebräuchlichen Glasfritte, eine gute Haftung des Materials auf der Keramik gewährleistet werden.

Aufgrund seines großen Meßbereiches (bis 25 Vol% CO₂) ist der Sensor für ein weites Feld von Anwendungen geeignet.

### Erzeugte Verbesserungen und Vorteile gegenüber dem Stand der Technik

Es ist gelungen, einen Metalloxid-Leitfähigkeitssensor für die Detektion von Kohlendioxid zu entwickeln. Der Sensor reagiert reversibel mit einer Widerstandsänderung auf eine Veränderung des CO₂-Anteils in der umgebenden Atmosphäre. Dieser Sensor ist in einem großen Meßbereich zur Bestimmung der CO₂-Konzentration einsetzbar.

Bei dem CO₂-Metalloxid-Leitfähigkeitssensor wird der Sensorwiderstand als Funktion der CO₂-Konzentration ausgewertet. Dies bietet die Möglichkeit, den Sensor als einfache Struktur aufzubauen. Es wurden zwei Interdigitalstrukturen als Elektroden eingesetzt, auf die das Sensormaterial aufgebracht wurde.

Aufgrund der gewählten Sensormaterialien und der einfachen Struktur des Sensors können zur Fertigung der Sensoren die Vorteile der Dickschichttechnik genutzt werden. Mit der Dickschichttechnik lassen sich reproduzierbare Sensoren herstellen: sowohl die Elektroden als auch die Sensorfläche werden im Siebdruckverfahren gedruckt. Eine rationelle Fertigung des Sensors ist somit gegeben.

Die Heizstruktur, welche zur Erzielung der notwendigen Arbeitstemperatur erforderlich ist, wird auf die Unterseite des Sensorkörpers gedruckt. Durch die direkte Ankopplung der Heizstruktur an das Substrat wird eine gute Wärmeübertragung erzielt und folglich ist ein geringer Energieaufwand zur Betreibung des Sensors erforderlich. Da die Temperatur an den Anschlußpads im Betriebsmodus trotz einer Sensortemperatur von bis zu 650°C nur ca. 150°C beträgt, können Kontaktierungen mittels Löt-, Bond- oder Klemmverbindungen ohne Weiteres realisiert werden.

Durch die Wahl der Elektrodenform, zwei Interdigitalstrukturen, die direkt auf das Substrat gedruckt werden, kann die aktive Schicht im letzten Schritt des Druckvorganges aufgebracht werden und wird somit von keiner weiteren Schicht bedeckt. Dies bedeutet, daß die gesamte Oberfläche des Metalloxid-Materials als aktive Schicht zur Verfügung steht.

Auf die üblicherweise für Haftzwecke in der Dickschichttechnik eingesetzten Glasfritte konnte durch die erfindungsgemäße Auswahl der Stoffe für die aktive Sensorschicht verzichtet werden. Das Sensormaterial haftet dennoch gut und weist gleichzeitig eine hohe Porosität auf. Infolge der relativ geringen Schichtdicke wird ein günstiges Verhältnis zwischen Sensorvolumen und Sensoroberfläche erzielt. Da sich die Effekte vorwiegend an den Komgrenzen abspielen, erhöht sich somit die Empfindlichkeit des Sensors, wobei sich die Ansprechzeit verringert.

Die einfache Sensorstruktur bietet gleichzeitig die Möglichkeit, den Sensorinnenwiderstand deutlich zu minimieren.

Da das Sensorprinzip auf einer Änderung der Leitfähigkeit des Metalloxides infolge einer Erhöhung bzw. Verringerung der Defektelektronen-Konzentration beruht, welche durch die Chemisorption des CO₂ an der Sensoroberfläche hervorgerufen wird, ist nur ein geringer meßtechnischer Aufwand erforderlich ist, um eine Bestimmung der CO₂-Konzentration durchzuführen. Die Auswertung einer Widerstandsänderung kann wesentlich einfacher und unkomplizierter realisiert werden als z.B. eine kapazitive Sensorauswertung.

Die hohe Arbeitstemperatur von ca. 570°C reduziert die Wasserquerempfindlichkeit.

Durch die gegebene Möglichkeit, mehrere verschiedene Sensoren auf einem Substratträger geringer Größe zu einer Sensormatrix zu vereinen, können Störeinflüsse kompensiert werden.

Angesichts der guten Reproduzierbarkeit, der geringen Investitionskosten, der Wirtschaftlichkeit, der Vielseitigkeit im Sensordesign, der Möglichkeit einer Miniaturisierung, der einfachen Herstellung der Pasten im Labormaßstab sowie der Einfachheit dieses Technologieverfahrens sind beste Voraussetzungen für eine Massenproduktion der CO₂-Metalloxid-Leitfähigkeitssensoren gegeben. Da die Herstellungskosten pro CO₂-Sensor sehr gering sind, ist eine breite Verfügbarkeit möglich.

### Grundzüge des Lösungsweges

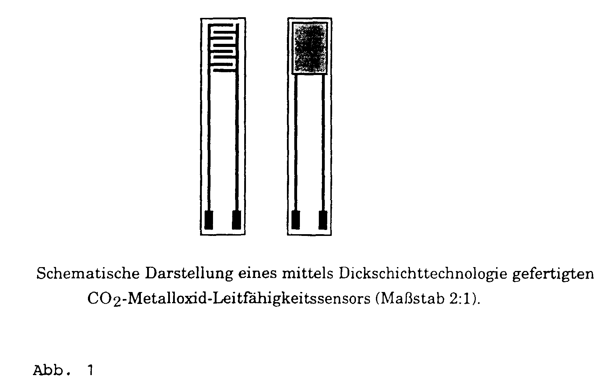

Der prinzipielle Aufbau des neu entwickelten CO₂-Metalloxid-Leitfähigkeitssensors ist in Abbildung 1 dargestellt. Der Sensorgrundkörper besteht aus Aluminiumoxid-Keramik. Zwei Interdigitalelektroden aus Gold wurden auf dessen Vorderseite gedruckt. Eine auf die Rückseite der Keramik gedruckte Heizstruktur ermöglicht die Einstellung der notwendigen Arbeitstemperatur.

Als Ausgangsmaterialien für die Herstellung der CO₂-empfindlichen Metalloxid-Paste wurden CuO und BaTiO₃ verwendet. Körner beider Materialien wurden zunächst getrennt voneinander in einer Planetenmühle gemahlen, um Komgrößen von kleiner 5µm zu erhalten. Diese Stoffe wurden im Anschluß an den Mahlvorgang in einem bestimmten Verhältnis mit weiteren Metalloxiden, die als Katalysator und als Haftvermittler dienen, vermengt.

Um eine Dickschichtpaste zu erhalten, ist die Zugabe von organischem Binder, organischen Lösungsmitteln und Glasfritte notwendig. Glasfritte bewirken jedoch ein dichtes Verschließen der Sensoroberfläche. Für die Bestimmung der CO₂-Konzentration ist es aber notwendig, eine möglichst große Oberfläche des Probenkörpers zur Verfügung zu haben. Aus diesem Grund wird auf Glasfritte verzichtet und die Haftfestigkeit des Sensormaterials auf der Al₂O₃-Keramik durch die Wahl der Metalloxide gewährleistet.

Als zusätzliche Metalloxide können z.B. La₂ O₃, Zr O₂, V₂ O₅, verwendet werden. Bevorzugt ist eine Mischung aus Cuₓ Cey O_{z}, wobei Cu und CeO₂ unter Anwesenheit von Sauerstoff auf eine Unterlage gesputtert werden und der Belag fein gemahlen wird. Die Bestandteile x, y, z bewegen sich im Bereich von etwa 1 - 5 und die Zahlen brauchen nicht ganzzeilig zu sein.

Das Pastengemisch wird in eine Planetenmühle gegeben, um eine homogene Durchmischung dieser Stoffe zu erzielen. Mittels Siebdrucktechnik wird die fertige Paste auf die Interdigitalstrukturen aufgetragen. Bei dem sich anschließenden Trocknen werden die flüchtigen organischen Lösungsmittel aus der Paste entfernt. Das Einbrennen der Paste erfolgt in zwei Schritten. Zunächst werden die organischen Pastenanteile herausgebrannt. Nach einer weiteren Temperaturerhöhung kommt es zu dem eigentlichen Sintervorgang.

Zur Fertigstellung des Sensors werden Drähte an die Pads gelötet und somit die Kontaktierung gewährleistet.

Die Sensortemperatur wird auf einen bestimmten Wert eingestellt.

Die Untersuchungen wurden an einem rechnergesteuerten Meßplatz durchgeführt. Als Spül- bzw. Trägergas wird ein Stickstoff-Sauerstoff-Gemisch verwendet, das in seiner Zusammensetzung der künstlichen Luft entspricht. Diesem Trägergas können verschiedene Prüfgase in selbst zu wählenden Konzentrationen beigemischt werden. Die Meßkammer wird zunächst mit künstlicher Luft gespült. Wird der Anteil des CO₂ erhöht, so kommt es zu einer Zunahme des Sensorwiderstandes. Eine Verringerung der CO₂-Konzentration läßt den Sensorwiderstand wieder sinken.

Dieser Effekt beruht auf folgendem Mechanismus: Der an der Metalloxid-Oberfläche adsorbierte Sauerstoff agiert als Oberflächenakzeptor und entnimmt dem Valenzband Elektronen. Dies führt zu einem Anwachsen der Ladungsträger-Konzentration (Defektelektronen). Da es sich bei dem vorliegenden Material um ein Gemisch mit einem großen Anteil an gleitenden Metalloxiden handelt, wird somit die Leitfähigkeit erhöht. Durch Zugabe von Kohlendioxid in die den Sensor umgebende Atmosphäre kommt es zu Reaktionen zwischen dem adsorbierten Sauerstoff und dem CO₂. Es folgt eine Verringerung der Oberflächenbelegung mit Sauerstoff und eine Abgabe von Elektronen an das Sensormaterial. Dies führt zu einer Verringerung der Ladungsträger-Konzentration und folglich zu einem Anwachsen des Sensorwiderstandes. Diese Oberflächenreaktionen werden wiederum durch das konzentrations- und katalysatorabhängige Gleichgewicht zwischen Adsorption und Desorption bestimmt, so daß letztendlich die Leitfähigkeit von der CO₂-Konzentration abhängig ist.

Es besteht ein logarithmischer Zusammenhang zwischen der Konzentrationsänderung des Kohlendioxids und der Änderung des Sensorwiderstandes.

### Ausführungsbeispiel

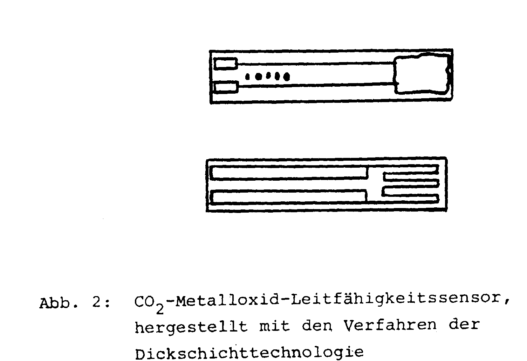

Der entwickelte CO₂-Metalloxid-Leitfähigkeitssensor (Abb. 2) wird mit den Verfahren der Dickschichttechnologie hergestellt.

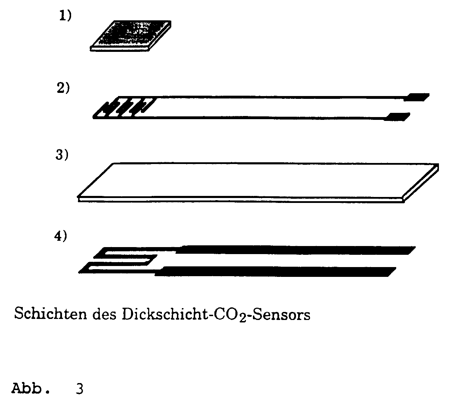

Ein Aluminiumoxid-Keramiksubstrat (3) bildet den Sensorgrundkörper (Abb. 3). Auf der Unterseite befindet sich die Heizstruktur (4), mit der die notwendige Arbeitstemperatur des Sensors eingestellt werden kann. Auf die Oberseite des Keramiksubstrates wurden die Interdigitalelektroden (2) aus Gold gedruckt. Diese Elektroden werden von dem Sensormaterial (1) bedeckt.

Die Dickschicht aus dem Sensormaterial ist nach der Sinterung etwa im Bereich von 20 - 200 µm, wobei das Material in Komgrößen von 1 nm - 400 nm vorliegt.

### Literatur

1. Maruyama, T., Sasaki, S., Saito, Y.: Potentiometric Gas Sensor for Carbon Dioxide Using Solid Elektrolytes. *Solid State lonics* 23:107-112, 1987.
2. Maruyama, T., Ye, X.-Y. , Saito, Y.: Electromotive Force of the CO-CO₂-O₂ Concentration Cell Using Na₂CO₃ as a Solid Electrolyte at Low Oxygen Partial Pressures. *Solid State lonics* 23:1 13-1 17, 1987.
3. Saito, Y., Maruyama, T.: Recent Developments of the Sensors for Carbon Oxides Using Solid Electrolytes. *Solid State lonics* 28-30:1644-1647, 1988.
4. Brandenburg, A., Edelhäuser, R., Hutter, F.: Integrated Optical Gas Sensors Using Organically Modified Silicates as Sensitive Films. *Sensors and Actuators B* 11:361-374, 1993.
5. Morgan, Ch.H., Cheung, P.W.: An Integrated Optoelectronic CO₂ Gas Sensor. *Digest of Technical Papers, Transducers '91, IEEE* 343-346, 1991.
6. Zhou, R., Vaihinger, S., Geckeler, K.E., Göpel, W.: Reliable CO₂ Sensors with Silicon-Based Polymers on Quartz Microbalance Transducers. *Sensors and Actuators B* 18-19:415-420, 1994.
7. Nieuwenhuizen, M.S., Nederlof, A.J.: A SAW Gas Sensor for Carbon Dioxide and Water. Preliminary Experiments. *Sensors and Actuators B* 2:97-101, 1990.
8. Mecea, V.M.: Tunable Gas Sensors. *Sensors and Actuators B* 15-16:265-269, 1993.
9. Lin, J., Heurich, M., Schlichting, V., Obermeier, E.: Characterization and Optimization of a CO₂-Sensitive Organically-Modified Silicate with Respect to its Use as a Gas Sensor. *Sensors and Actuators B* 13-14:528-529, 1993.
10. Heurich, M., Lin, J., Schlichting, V., Obermeier, E.: CO₂-Sensitive Organically Modified Silicates for Application in a Gas Sensor. In: Micro-system technologies 92. Reichl, H. (ed.) Berlin, Offenbach. VDE Verlag GmbH. 1992. p. 359-367.
11. Ishihara, T., Kometani, K., Mizuhara, Y., Takita, Y.: Mixed Oxide Capacitor of CuO-BaSnO₃ as a Sensor for CO₂ Detektion over a Wide Range of Concentration. *Chemistry Letters* 171 1-1714, 1991.
12. Ishihara, T., Kometani, K., Hashida, M., Takita, Y.: Mixed Oxide Capacitor of BaTiO₃-PbO as a New Type CO₂ Gas Sensor. *Chemistry Letters* 1163-1166, 1990.
13. Ishihara, T., Kometani, K., Mizuhara, Y., Takita, Y.: Capacitive Type Gas Sensor for the Selective Detection of Carbon Dioxide. *Technical Digest of the Fourth International Meeting on Chemical Sensors, Tokyo* 538-541, 1992.
14. Ishihara, T., Kometani, K., Mizuhara, Y., Takita, Y.: Capacitive-Type Gas Sensor for the Selective Detection of Carbon Dioxide. *Sensors and Actuators B* 13-14:470-472, 1993.
15. Tränkler, H.-R.: Gassensorik heute und morgen. *Sensor report* 1:32-39,1993.
16. Hermans, E.C.M.: CO, CO₂, CH₄ and H₂O Sensing by Polymer Covered Interdigitated Electrode Structures. *Sensors and Actuators* 5:181-186, 1984.

## Patentansprüche

1. Chemischer halbleitender Festkörper-Sensor mit einem Keramikträger (3), auf dem auf der Unterseite ein Heizelement (4) mit Leitungsanschlüssen aufgebracht ist und auf der Oberseite Interdigitalelektroden (2) und darauf ein für CO₂ gassensitives Material (1) aus CuO und Ba-TiO₃ angeordnet sind und die Leitfähigkeit mittels Widerstandsmessung in Abhängigkeit von der Konzentrationsänderung des CO₂ gemessen wird,
**dadurch gekennzeichnet,**
daß zusätzlich Metalloxide als Katalysatoren und/oder Haftvermittler vorgesehen sind, wobei als Metalloxid Cₓ Ce_{y} Q_{z} verwendet wird, mit x, y, z im Bereich von etwa 1 - 5, wobei x y, z nicht ganzzahlig sein müssen, und der Sensor in gesinterter Form eine Schichtdicke von etwa 20 - 200 µm aufweist.

2. Sensor nach Anspruch 1,
**dadurch gekennzeichnet,**
daß Material in Korngrößen von < 5 µm, vorzugsweise im Nanostrukturbereich von 1 nm - 400 nm vorgesehen ist.

3. Sensor nach Anspruch 1 oder 2,
**dadurch gekennzeichnet,**
daß Katalysatoren Bestandteil des Materials sind, mit einem Anteil von 0,1 - 10 %.

4. Sensor nach Anspruch 1,
**dadurch gekennzeichnet,**
daß die Interdigitalelektroden aus Gold oder einem anderen Edelmetall bestehen und die Heizelektroden aus Platin oder Palladium.

## Claims

1. Chemical semi-conducting solid state sensor with a ceramic carrier (3), onto the underside of which a heating element (4) with circuit connections is deposited and on the top-side of which there are interdigital electrodes (2) and on top of which is arranged a material (1), which is made of CuO and Ba-TiO₃ and which is sensitive to CO₂ and the conductivity of which is measured by means of resistance measurement which is dependent upon the change in concentration of CO₂,
characterised in that,
there are additional metal oxides used as catalysts and/or adhesive conveyors, CₓCe_{y}O_{z} being used as a metal oxide in the range of approx. 1-5, where x, y, and z need not be whole numbers and where the sensor has a coating thickness of approx. 20-200µm in its sintered form.

2. Sensor according to Claim 1,
characterised in that,
there is material with particles of < 5µm, preferably in the nanostructure range of 1nm-400nm.

3. Sensor according to Claims 1 or 2,
characterised in that,
catalysts are a component of the material, having a proportion of 0.1-10%.

4. Sensor according to Claim 1,
characterised in that,
the interdigital electrodes consist of gold or another noble metal and the heating electrodes are made of platinum or palladium.

## Revendications

1. Détecteur chimique à corps solide semi-conducteur avec un support céramique (3), sur lequel on met sur la face inférieure un élément de chauffage (4) avec des raccordements de ligne et on dispose sur sa surface des électrodes interdigitales (2) et dessus une matière (1), sensible aux gaz pour le CO₂, matière qui consiste en un oxyde de cuivre CuO et un oxyde de Baryum et de titane Ba-TiO3, et l'on mesure sa conductibilité au moyen d'une mesure de sa résistance en fonction des variations de la concentration du CO₂,
caractérisé en ce que
l'on prévoit en plus des oxydes métalliques comme catalyseurs et/ou agent d'adhérence, en utilisant comme oxyde métallique Cx Cey Oz, avec x, y, z se trouvant dans la zone d'environ 1 à 5, x, y, z n'ayant pas besoin d'être des nombres entiers, et le détecteur présentant dans sa forme frittée une épaisseur de couche d'environ 230 à 200 µm.

2. Détecteur selon la revendication 1,
caractérisé en ce que
l'on prévoit que la matière ait une grosseur de grains inférieure à 5 µm, et de préférence se trouve dans la zone de nanostructure de 1 nm à 400 nm.

3. Détecteur selon la revendication 1 ou 2,
caractérisé en ce que
les catalyseurs sont des constituants de la matière avec une proportion de 0,1 à 10 %.

4. Détecteur selon la revendication 1,
caractérisé en ce que
les électrodes interdigitales sont en or ou en un métal précieux et les électrodes de chauffage sont en platine ou en palladium.
